# EUROPEAN PATENT APPLICATION

(11) **EP 4 527 944 A1**
(43) Date of publication of application: **26.03.2025**
(21) Application number: 23199177.9
(22) Date of filing: 22.09.2023
(51) Int. Cl.: C12Q 1/6883, C12Q 1/689, G01N 33/68

(54) **GUT MICROBIOTA OLSENELLA UMBONATA AS BIOMARKER FOR EARLY DETECTION OF ALZHEIMER'S DISEASE**

(71) Applicant: Vilnius University, 01513 Vilnius (LT)
(72) Inventor: Burokas, Aurelijus, Vilnius (LT); Atzeni, Alessandro, Vilnius (LT); Daliri, Eric Banan-Mwine, Vilnius (LT); Baltriukiene, Daiva, Vilnius (LT); Mingaila, Jonas, Vilnius (LT); Vijaya, Akshay Kumar, Vilnius (LT)
(74) Representative: Petniunaite, Jurga

(57) **Abstract**

The present invention relates to a Gram-positive, anaerobic and non-spore-forming bacterium in the stool that can serve as a biomarker for diagnosing, assessing and predicting the development of dementia related diseases induced by high fat diet consumption. This was done by analyzing bacterial metagenome using cecum samples derived from AD mice models and to investigate the increase of a specific bacterium's relative abundance and its association with the disease. By using the strategy in this invention, it is possible to diagnose and predict AD at an early stage, so that it is possible to delay the onset of the disease or to prevent the onset of the disease through proper management thereby lowering the incidence of AD and improving the therapeutic effect.

## Description

### FIELD OF THE INVENTION

The present invention relates to the field of microbiology, bioinformatics, molecular biology and specifically to dementia, especially an Alzheimer's disease, biomarker and application thereof.

### BACKGROUND OF THE INVENTION

A rapid population ageing has resulted in a growing number of patients with Alzheimer's disease (AD) in Europe that creates an increasing need for early diagnosis, treatment, and prevention of illness [1]. Additionally, the growing "epidemic" of diabetes and its close relationship with AD is increasingly becoming more evident that led researchers to investigate how these diseases have an influence on each other [2]. In both cases, impaired immune system that is closely related to the gut microbiota has been reported [2].

Gut microbiome (analyzed in cecum and/or feces) influences gastrointestinal and brain disorders such as Alzheimer's disease (AD). Neuroinflammation, tau hyperphosphorylation, amyloid plaques, neurofibrillary tangles, and oxidative stress, have been proposed as potential causes for the origin and progression of AD. However, the pathogenesis of AD remains incompletely understood. Epigenetic, molecular, and pathological studies suggest that gut microbiota influences AD development, highlighting the need of predictive, sensitive, non-invasive, and accurate gut biomarkers identification for both preclinical and clinical diagnoses [3].

Gut microbial imbalance have been observed during the initial stages of AD and, conversely, AD pathophysiological mechanisms, such as the activation of microglia and Aβ assimilation, shown to disrupt the intestinal microbiome balance [4]. AD patients are associated with distinct microbial signatures, with an increase in pathogenic bacteria and reduced levels of beneficial bacteria. Thus, the restoration of the microbiota could reduce neurodegeneration and improve cognitive function among AD patients. Moreover, distinctive gut microbial profiles could aid in the risk estimation of AD [4].

Type 2 diabetes mellitus is a commonly observed comorbid condition among AD patients. Furthermore, immunological dysfunction and impaired glucose, amyloid, and insulin metabolism have been observed among diabetic and AD patients [4].

With the growing number of facts about the effects of the microbiota on various physiological processes in the body, its exploration has been chosen not only to better understand the mechanism of AD progression, but also to use it as a biomarker in order to create a method for early diagnosis of the disease, which would enable doctors to start treatment much earlier. In genetically modified mice models od AD, 16S gene sequencing revealed specific bacteria that might be important biomarkers of AD [5]. Moreover, significant changes in gut microbial composition are observed, especially in older people, and diet may be the most important driver of change [6].

The early identification of AD pathology traits is fundamental for the diagnosis, disease monitoring, and management of AD patients. Currently, the available biomarkers for early diagnosis of AD are mostly represented by blood-based biomarkers [7]. However, many pharmacological treatments targeting Aβ and tau pathological features showed limitations probably attributed to the difficulty of reversing the underlying neuropathological changes that have already occurred. Therefore, very early and reliable diagnostic biomarkers are urgently needed. Non-invasive biomarkers such as stool, saliva, or urine may be a realistic option for large-scale population screening, early AD diagnosis, and disease progression monitoring at a low cost [4].

A number of recent research publications have investigated the possibility of identifying gut bacteria that may serve as biomarkers of AD and its associated diseases [8-11]. Meanwhile, to the best of our knowledge, none of them has reported a strong associated between AD and a specific bacterium specie that could serve as a potential biomarker for early diagnosis of AD claimed in this invention. Prior documents rather report a consortium of bacteria or an entire genera as potential indicators of the disease. It is however laborious and almost impossible to develop primers to detect all the species of different genera that are claimed to be associated with AD. Identifying a specific specie that can be easily detected and quantified such as reported in this invention is necessary for easy detection. More so, some other inventions closely related to the current invention sought for fecal metabolic biomarkers of AD but not fecal bacteria that are associated with the disease (CN115791987A, CN115791986A, WO2023035465A1 and WO2023039954A1). The weakness of other inventions is that they failed to account for which gut microbe or microbes that may have generated the metabolites. Detecting the metabolites without knowing their sources therefore do not give clues how the gut microbiota must be modulated to halt the disease progression. This invention however identifies a specific bacterium which can serve as a biomarker for early detection and control of its potential toxic metabolites that could play a role in AD progression.

The closest analogue to the invention is described by Y. Li et al. (Dynamic network modeling of gut microbiota during Alzheimer's disease progression in mice, Gut microbes 15(1) (2023) 2172672.https://doi.org/10.1080/19490976.2023.2172672) who identified bacteria family *Lachnospiraceae* UCG-001 as a microbial biomarker of AD. The similarities lie in the fact that both art use AD mice models, monitor the progression of AD and observe severe gut microbiota dis-equilibrium at the late stage of the disease. Both arts observe close associations between increasing populations of certain gut bacteria and disease severity.

Meanwhile, the differences between the inventions are clear since the prior art identifies a higher taxonomic rank (a bacteria family) as the marker of the disease while the current invention identifies a specific bacterium species as the biomarker of the disease.

### Similar Documents

| Publication | Publication Date | Title |
|---|---|---|
| Cammann, et al. | 2023 | Genetic correlations between Alzheimer's disease and gut microbiome genera |
| Wanapaisan et al. | 2023 | Association between Gut Microbiota with Mild Cognitive Impairment and Alzheimer's Disease in a Thai Population |
| Borsom et al. | 6 March 2023 | Predicting neurodegenerative disease using prepathology gut microbiota composition: a longitudinal study in mice modeling alzheimer's disease pathologies |
| Li et al. | 1 Feb 2023 | Dynamic network modeling of gut microbiota during Alzheimer's disease progression in mice |
| CN115791987A | 2023-03-14 | Alzheimer's disease biomarker inosine and application thereof |
| CN115791986A | 2023-03-14 | Alzheimer's disease biomarker L-valine and application thereof |
| WO2023039954A1 | 2023-03-23 | S-methyl-5'-thioadenosine biomarker for Alzheimer's disease, and use thereof |
| WO2023035465A1 | 2023-03-16 | Taurine as biomarker for Alzheimer's disease and use thereof |

### SUMMARY OF THE INVENTION

The present invention relates to gut bacteria *Olsenella umbonata* in the cecum that can be used as biomarkers of dementia and its associated diseases including Alzheimer's disease. In particular, embodiments of the invention relate to the surprising overabundance of a Gram-positive, anaerobic and non-spore-forming bacterium for diagnosing, assessing and predicting the development of dementia related diseases induced by high fat diet consumption.

The present inventors extracted bacteria DNA from cecum contents obtained from AD mice models and control mice in order to diagnose the causative factors of diabetes, risk of onset, and disease progression. The inventors completed the present invention by identifying bacterial that were associated with AD and are capable of acting as indicators of the disease condition.

To achieve this, 8-week-old C57BI/6J inbred male and female mice (n=91) were either fed with normal rodent diet or exposed to diet-induced diabetes to provoke neuro-inflammatory processes related to the development of AD. At 15 months, several behavioral experiments were performed to confirm AD.

Cecum contents were collected from the mice and bacteria DNA was extracted for shotgun sequencing.

This summary of the invention does not necessarily describe all features of the invention.

### DESCRIPTION OF DRAWINGS

The drawings are provided as a reference to possible embodiments and are not intended to limit the scope of the invention. Neither of the drawings nor the graphs presented herein should be construed as limiting the scope of the invention, but merely as an example of a possible embodiment.

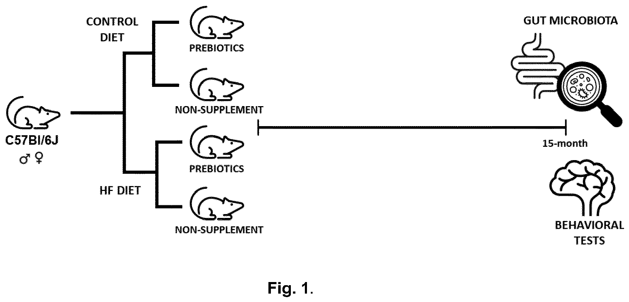

Fig. 1. Schematic representation of the study conducted. 91, 8-week-old C57BI/6J inbred male and female mice were assigned to each diet (high-fat or to the control group), with ad libitum access food. Additionally, these diet groups were divided into prebiotic supplemented group, and non-supplemented group. Caecum content for bacterial DNA isolation, and shotgun sequencing, was collected at 15 months. In addition, behavioral tests indicative of AD progression were also conducted at 15 months.

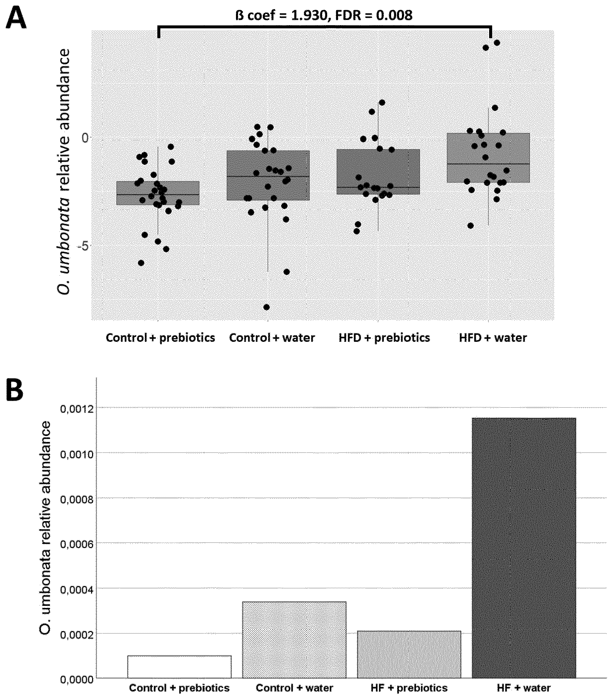

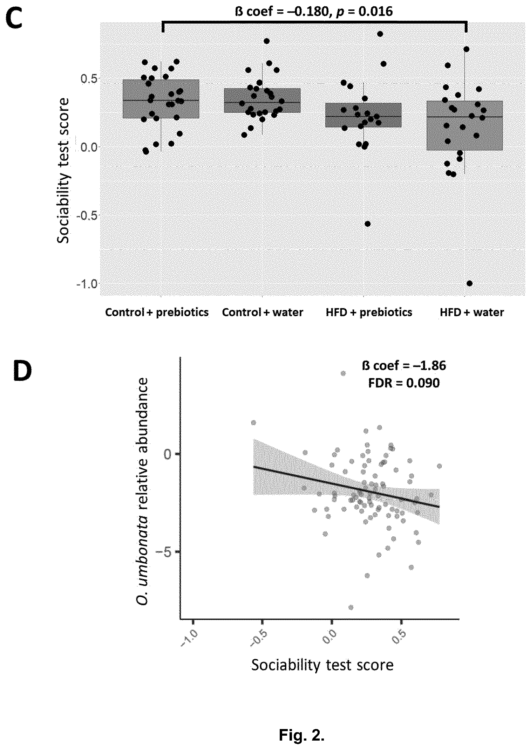

Fig. 2. Resume of results obtained. Higher abundance of *Olsonella umbonata* was observed in HF non-supplemented group compared with control supplemented group (A); the total relative abundance of *O. umbonata* in HF non-supplemented group was 0.0012 (66.67%), whereas the total abundance in control supplemented group was 0.0001 (5.5%) (B); the sociability test score was lower in HF non-supplemented group compared with control supplemented group (C); A negative association between *O. umbonata* and the sociability test score was observed (D). All the statistics were conducted through linear model wherein *p* < 0.5, and FDR < 0.1 was deemed significant.

### DETAILED DESCRIPTION OF THE INVENTION

To further illustrate the technical means adopted by the present invention and the effects thereof, the present invention is further described below with reference to the embodiments and the accompanying drawings. It is to be understood that the specific embodiments described herein are merely illustrative of the invention and are not limiting of the invention.
a) In order to determine the microbial biomarker of a AD, a group of mice representative of the health state and AD group of were developed by feeding 8-week-old C57BI/6J inbred mice (n=91) with either normal rodent diet or high fat diet (HFD). At 15 months, the mice were assessed for behavioral patterns indicative of AD. Sociability and preference for social novelty Social behavior was performed in the V-social-maze (30 cm long × 4.5 cm wide × 15 cm height each corridor). Briefly, the protocol consists of three phases: habituation (Phase I), sociability (Phase II), and preference for social novelty (Phase III). First, experimental mice were introduced into the central part of the V-maze where they freely explored the two empty chambers at the end of the corridors. This measurement is important to discard a possible bias for one particular chamber and it informs about the baseline activity of the mouse in the maze. Then, during the sociability phase an unfamiliar juvenile mouse assigned as stranger 1, was placed in one of the chambers (both sides were alternated during the experiments). The experimental mouse was allowed to explore both compartments for 5 min. The experimenter recorded the time that the experimental mouse spent exploring the empty chamber or the stranger 1. Finally, the preference for social novelty phase was performed just after the sociability session. A second novel juvenile mouse, assigned as stranger 2, was placed inside the previously empty chamber, while the stranger 1 remained inside the same chamber as in Phase II. For 5 min, the experimental animal was allowed to explore the two strangers and the time spent exploring each stranger was recorded. The procedure was performed in a sound-attenuated room with dim illumination 5-10 lux. A digital camera on top of the maze was used to record the sessions. Social exploration was considered when the experimental mouse directed the nose in close proximity (1 cm) to the vertical bars of the chambers. Mice that explored <10 s both mice were excluded from the analysis. Mice that were fed with HFD displayed characteristics of AD as expected. Cecum samples were collected from the AD mice models were compared to the same samples taken from the control group in the hopes of identifying microbial differences between the two groups, by analyzing the bacteria present in the samples using next generation amplicon sequencing.
b) Caecum samples were thawed, weighed, diluted in sterile phosphate buffer solution and homogenized in a sterile cabinet. Bacterial pellet and faecal water were separated by centrifugation and kept for further analyses: DNA isolation and chromatography analyses, respectively. Bacterial DNA was isolated using QIAamp Fast DNA Stool Mini Kit (Qiagen; Düsseldorf, Germany) following the manufacturer's protocol, with some modifications and including a bead-beating step in a Fastprep (MP Biomedicals, Thermo Fisher Scientific Inc.).
c) The Whole genome mWGS sequencing was performed using Illumina platform, with paired-end reads (PE150 Q30≥80%). The genomic DNA was randomly sheared into short fragments. The obtained fragments were end repaired, A-tailed and further ligated with Illumina adapter. The fragments with adapters were PCR amplified, size selected, and purified. The library was checked with Qubit and real-time PCR for quantification and bioanalyzer for size distribution detection. Quantified libraries were pooled and sequenced on Illumina platform. Raw Data contained a certain percentage of low-quality data after sequencing. To ensure the accuracy and reliability of the subsequent information analysis, quality control and host filtering of the raw data were carried out to obtain clean data.
d) For metagenomics assembly, samples that passed quality control were assembled using MEGAHIT. Clean data of all samples were mapped to assembled Scaftigs using Bowtie2 and unutilized PE reads were collected and trimmed. The effective Scaftigs were used for further analysis and gene prediction. The gene prediction was carried out by MetaGeneMark based on the Scaftigs which were assembled by single and mixed samples. Then a pooling of the predicted genes together for dereplication to construct gene catalogue was carried out. Based on the clean data of each sample from the gene catalogue, the abundance information of the gene catalogue for each sample was obtained.
e) Taxonomic analysis was carried out by comparing metagenomic reads to the database of taxonomically informative gene families (microNR database) to annotate each metagenomic homolog (MEGAN). The abundance tables of different taxonomic ranks were based on gene abundance table.
f) Statistical analyses based on linear models were conducted to test the association between the different diets and the behavioral tests scores, and the association between different diets and specific gut microbiota features abundances. Finally, the association between diet-related gut microbiota features and diet-related behavioral tests scores was assessed to identify potential bacteria involved in the modulation of AD.
g) Among the diet-related gut microbiota features, the inventors observed higher abundance of *Olsonella umbonata* in HF non-supplemented group compared with control supplemented group (coef = 1.930, p-value = 0.0003, FDR = 0.0078) (Figure 2A).
h) The inventors also observed significant difference in sociability between the same study groups: it was lower in HF non-supplemented group compared with control supplemented group (Figure 2B). Accordingly, we tested the association between *O. umbonata* abundance and sociability test, observing a negative association between this specie and the test score (Figure 2C). Al the statistics were conducted through linear model and FDR < 0.1 deemed significant.

### References

[1] G. Livingston, J. Huntley, A. Sommerlad, D. Ames, C. Ballard, S. Banerjee, C. Brayne, A. Burns, J. Cohen-Mansfield, C.J.T.L. Cooper, Dementia prevention, intervention, and care: 2020 report of the Lancet Commission, 396(10248) (2020) 413-446. https://doi.orp/10.1016/S0140-6736(20)30367-6
[2] J.A. Luchsinger, D.R.J.J.o.A.s.D. Gustafson, Adiposity, type 2 diabetes, and Alzheimer's disease, 16(4) (2009) 693-704. https://doi.org/10.3233/JAD-2009-1022
[3] L. Krishaa, T.K.S. Ng, H.N. Wee, J.J.M.o.A. Ching, Development, Gut-brain axis through the lens of gut microbiota and their relationships with Alzheimer's disease pathology: review and recommendations, (2023) 111787. https://doi.org/10.1016/j.mad.2023.111787
[4] Y. Zhan, M. Al-Nusaif, C. Ding, L. Zhao, C.J.F.i.N. Dong, The potential of the gut microbiome for identifying Alzheimer's disease diagnostic biomarkers and future therapies, 17 (2023) 1130730. https://doi.orp/10.3389/fnins.2023.1130730
[5] M. Feng, T. Hou, M. Zhou, Q. Cen, T. Yi, J. Bai, Y. Zeng, Q. Liu, C. Zhang, YJ.F.i.A.N. Zhang, Gut microbiota may be involved in Alzheimer's disease pathology by dysregulating pyrimidine metabolism in APP/PS1 mice, 14 (2022) 967747. https://doi.org/10.3389/fnagi.2022.967747
[6] M. Kumar, P. Babaei, B. Ji, J.J.N. Nielsen, H. aging, Human gut microbiota and healthy aging: recent developments and future prospective, 4(1) (2016) 3-16. https://doi.org/10.3233/NHA-150002
[7] Q.-Q. Tao, R.-R. Lin, Z.-Y.J.C.I.i.A. Wu, Early Diagnosis of Alzheimer's Disease: Moving Toward a Blood-Based Biomarkers Era, Taylor & Francis, 2023, pp. 353-358. https://doi.org/10.2147/CIA.S394821
[8] D. Cammann, Y. Lu, M.J. Cummings, M.L. Zhang, J.M. Cue, J. Do, J. Ebersole, X. Chen, E.C. Oh, J.L. Cummings, J. Chen, Genetic correlations between Alzheimer's disease and gut microbiome genera, Scientific Reports 13(1) (2023) 5258. https://doi.orp/10.1038/s41598-023-31730-5.
[9] P. Wanapaisan, M. Chuansangeam, S. Nopnipa, R. Mathuranyanon, N. Nonthabenjawan, C. Ngamsombat, T. Thientunyakit, W. Muangpaisan, Association between Gut Microbiota with Mild Cognitive Impairment and Alzheimer's Disease in a Thai Population, Neurodegenerative Diseases 22(2) (2023) 43-54. https://doi.ora/10.1159/000526947 %J Neurodegenerative Diseases.
[10] E.M. Borsom, K. Conn, C.R. Keefe, C. Herman, G.M. Orsini, A.H. Hirsch, M.P. Avila, G. Testo, S.A. Jaramillo, E. Bolyen, K. Lee, J.G. Caporaso, E.K. Cope, Predicting Neurodegenerative Disease Using Prepathology Gut Microbiota Composition: a Longitudinal Study in Mice Modeling Alzheimer's Disease Pathologies, 11 (2) (2023) e03458-22. https://doi.orq/doi:10.1128/spectrum.03458-22.
[11] Y. Li, Y. Chen, Y. Fan, Y. Chen, Y. Chen, Dynamic network modeling of gut microbiota during Alzheimer's disease progression in mice, Gut microbes 15(1) (2023) 2172672. https://doi.orp/10.1080/19490976.2023.2172672.

## Claims

1. A biomarker determining dementia being a gut bacteria **characterized in that** the gut bacteria are *Olsenella umbonate* bacteria originating in caecum and/or feces.

2. The biomarker according to claim 1, wherein the *Olsenella umbonate* bacteria are gram-positive, anaerobic and non-spore-forming bacterium.

3. The biomarker according to claim 1 or 2, where the *Olsenella umbonate* bacteria are extracted from caecum or feces as bacterial DNA samples.

4. The biomarker according to any one of the preceding claims, where abundance of *Olsonella umbonata* bacteria in high-fat non-supplemented group compared with control supplemented group is coef = 1.930, p-value = 0.0003, FDR = 0.0078.

5. A method for obtaining biomarker determining dementia according to any one of claims 1-4, **characterized in that** the amount of *Olsenella umbonate* bacteria in caecum or feces is adjusted by administration of high-fat diet.

6. The method according to claim 5, where the *Olsenella umbonate* bacteria are used for assessment of dementia after 15 months after administration of high-fat diet.

7. The method of claim 6, where samples of *Olsenella umbonate* bacteria are thawed, weighed, diluted in sterile phosphate buffer solution and homogenized in a sterile cabinet, bacterial pellet and faecal water, separated by centrifugation and analyzed by generation amplicon sequencing, comprising:
DNA isolation and chromatography analyses, respectively,
taxonomic analysis carried out by comparing metagenomic reads to the database of taxonomically informative gene families, and
statistical analyses conducted.

8. Use of the biomarker of any one of claims 1-4 for determination Alzheimer's disease.
